(19) Europäisches Patentamt

European Patent Office

Office européen des brevets

(11) **EP 4 696 370 A1**

(12) **EUROPEAN PATENT APPLICATION**

(43) Date of publication:
**18.02.2026  Bulletin 2026/08**

(21) Application number: **24194442.0**

(22) Date of filing: **14.08.2024**

(51) International Patent Classification (IPC):
**A61N 1/378** (2006.01)　　**H02J 50/10** (2016.01)

(52) Cooperative Patent Classification (CPC):
**A61N 1/3787; H02J 50/10;** H02J 2105/46

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **BIOTRONIK SE & Co. KG
12359 Berlin (DE)**

(72) Inventors:
• **VON ARX, Jeffrey A.
Lake Oswego, 97035 (US)**
• **WHITTINGTON, R. Hollis
Portland, 97202 (US)**
• **MUESSIG, Dirk
West Linn, 97068 (US)**
• **FRYER, Alan
Portland, 97202 (US)**

(74) Representative: **Biotronik Corporate Services SE
Corporate Intellectual Property
Sieversufer 9
12359 Berlin (DE)**

(54) **WIRELESS CHARGER FOR AN IMPLANTABLE MEDICAL DEVICE**

(57)　A charger (20) for wirelessly charging an implantable medical device, IMD (1) that is implanted in a patient in tissue (72) under the skin (70). The charger (20) has an electrical energy source (22, 24) connected to power transmit coils (23) that when energized generate an electromagnetic field for wirelessly charging an implanted IMD (1). The charger is provided with an active cooling device (21) powered by the electrical energy source and arranged to cool a surface portion of the charger that is designed to be in contact with the patient's skin during wireless charging. Actively cooling the skin (70) under the charger (20) increases the temperature gradient between the skin (70) and the IMD (1), thereby increases the rate of heat dissipation from the IMD (1). The rate of charging can therefore be increased so that charging times are reduced.

**FIG. 2**

EP 4 696 370 A1

# Description

The invention relates to a charger for wirelessly charging an implantable medical device (IMD) implanted in a patient.

An IMD with on-board electronics will typically require an electrical power source in the form of a battery. If the IMD is implanted close to the skin, i.e., subcutaneously, which is typically the case for neurostimulation devices such as a spinal cord stimulator (SCS), it is convenient to fit a rechargeable battery (i.e. secondary cell) that is charged *in situ* with a suitable wireless charger that is placed on the skin by the patient or a caregiver over the IMD to provide for contactless charging. For this purpose, the IMD is provided with pick-up coils, referred to as receive coils, to allow the rechargeable battery to be wirelessly charged through inductive coupling to a matched set of coils in the charger, referred to as transmit coils.

While the use of a rechargeable battery allows a much smaller battery to be used compared with a non-rechargeable battery (i.e. primary cell), it self-evidently must be recharged from time-to-time and the responsibility for this task lies with the patient. Charging may be needed at intervals of a week or sometimes more frequently. Each charge may take thirty minutes, assuming the transmit and receive coils are well aligned. Charging times are substantially longer if the coils are misaligned.

In a conventionally constructed IMD for SCS there are two principal units: a header and a can. The header is the interface area for conductive leads for SCS which are embedded in a nerve trunk of the patient. These carry therapeutic stimulation pulses and also may be used for sensing evoked compound action potential (ECAP) signals. The header casing is typically of a plastics material or an epoxy. The can contains the electronics, sensors, and communications hardware, as required for external communication using a suitable wireless or near-field protocol. The can also contains the rechargeable battery. The can has a metal outer casing, made of a biocompatible metal, typically titanium or a titanium alloy.

Depending on the manufacturer, the IMD's receive coils are placed in the header, in the can, or in a separate ceramic housing brazed to the can.

The motivation for not accommodating the receive coils in the can is to reduce heating of the metal casing of the can that is caused by eddy currents generated by the time-varying magnetic field generated by the transmit coils of the charger. A significant amount of eddy current heating of the metal casing of the can does however still occur even when the receive coils are moved out of the can.

For safety, it is important to limit the heating of the metal outer casing that takes place during wireless charging, since it is in direct contact with the patient's tissue. The industry standard ISO 14708-3:2017 specifies temperature limits. The definition is based on a thermal dose limit expressed in cumulative equivalent minutes at 43°C (referred to as CEM43), this being approximately 6°C above normal body temperature. The CEM43 dose is defined by the following integral.

$$CEM43°C = \int_0^t d\tau R^{43-T(\tau)}$$

$$R = \begin{cases} 0.5 \ if \ T \geq 43°C \\ 0.25 \ if \ T \ < 43°C \end{cases}$$

As seen from the above formula, the CEM43 dose increases exponentially when the temperature exceeds 43°C. The choice of this value is made, since prolonged exposure to temperatures above 43°C causes tissue damage. The general aim when wireless charging is to maximize the charging rate of the IMD battery while keeping the thermal dose within the dose limit. To avoid overheating, it is known to provide a temperature sensor in the IMD to measure the temperature of the metal casing so that charging is paused, or at least charging power is reduced, whenever the metal casing becomes too hot, e.g., too close to 43°C, and then restarted, or increased, after the metal casing has cooled sufficiently, e.g., to a temperature having a certain delta below 43°C.

The rate of energy transfer across an inductive link is defined by a coupling coefficient, which depends on several parameters including the distance between the transmit and receive coils, the relative orientation between the coils, and the quality factor of the coils. For a given coupling coefficient, the rate at which energy is transmitted is limited by heating.

Inductive charging heats tissue in several ways. For most rechargeable IMDs the dominating heating modality is from eddy currents induced on the metal casing of the IMD can from the varying magnetic field generated by the transmit coil. Another significant heating modality is electro-chemical heating of the battery as it charges as well as eddy current heating of the battery outer jacket which is made of metal. Another significant heating modality is heating the tissue itself since tissue is quite conductive. Tissue will have a specific absorption rate (SAR) for the charging fields that results in the charging field directly heating the tissue. Since the strength of the varying magnetic field generated by the transmit coils drops with distance away from the transmit coil, areas of the metal casing that are closer to the charger will experience more heating than those that are further away. For the same reason, the skin and tissue closest to the skin will be heated more strongly than tissue further away. The tissue situated between the IMD and the charger is the tissue that is most strongly heated.

Controlling and reducing heating while wirelessly charging the IMD's rechargeable battery is therefore central to patient safety, patient comfort and reducing charging times.

According to first aspect of the disclosure there is provided a charger for wirelessly charging an implantable medical device, IMD, that is implanted in a patient, the charger comprising:

an electrical energy source;
transmit coils arranged to generate an electromagnetic field for wirelessly charging an implanted IMD; and
a cooling device powered by the electrical energy source and arranged to cool a surface portion of the charger that is configured to be in contact with the patient's skin during wireless charging.

Actively cooling the skin under the charger increases the temperature gradient between the skin and the IMD, specifically its metal casing and its receive coil. The increased temperature gradient increases the rate of heat dissipation from the IMD, which in turn reduces the temperature of the IMD during charging. The rate of charging can therefore be increased. The reduced charging time makes it more likely that the patient will stay compliant by charging the IMD whenever this is needed.

In some embodiments, the cooling device is a thermoelectric cooler operating by the Peltier effect, the thermoelectric cooler having a cold plate and a hot plate, the cold plate forming part of said charger surface portion. Cooling fins may be provided which are arranged to extract heat from the cooling device, e.g., in thermal contact with the hot plate of a thermoelectric cooler.

The charger may include one or more temperature sensors arranged to measure temperature of the cooling fins. The charger may include one or more temperature sensors arranged to measure temperature of said charger surface portion.

The charger may include a controller loaded with machine readable instructions to control the charging of an IMD. The IMD charging process may be controlled by the controller according to the machine-readable instructions dependent on the temperatures measured by the temperature sensors built into the charger. The charger may include a wireless transceiver operable to receive IMD temperature data from the IMD being charged, wherein the charging of the implanted IMD is controlled by the controller according to the machine-readable instructions dependent on the IMD temperature data. It is therefore possible to control the charging process taking account of various temperature readings from different locations in the charger and the IMD.

The electrical energy source may be selected from one or more of the group: rechargeable battery; non-rechargeable battery; and external electrical power connection.

This invention will now be further described, by way of example only, with reference to the accompanying drawings.

Fig. 1    is a schematic diagram of an IMD arranged in a medical device communication system (MDCS).

Fig. 2    is a schematic cross-sectional drawing of a charger embodying the invention in position to wirelessly charge an implanted IMD.

Fig. 3    is a schematic plan view in the proximal direction of the charger of Figure 2.

In the following detailed description, for purposes of explanation and not limitation, specific details are set forth in order to provide a better understanding of the present disclosure. It will be apparent to one skilled in the art that the present disclosure may be practiced in other embodiments that depart from these specific details.

In particular, the embodiments described in the following are described by way of example only in relation to an SCS system.

An SCS device is a type of IMD that is used for chronic pain therapy of a patient. An SCS device uses electrical current sent through electrodes on one or more leads implanted in the epidural space dorsal to the spinal cord in an SCS device. Control parameters include at least: pulse width, frequency, amplitude, and electrode selection.

An IMD typically forms part of a medical device communication system (MDCS) that is configured to upload clinical data from the IMD on a continual basis via a MDCS to a remotely located data repository, which may be a data center. In an SCS device, clinical data includes both patient data relating to physiological monitoring of a patient's health, for example as collected by sensors of the IMD, and device data related to status and operation of the IMD, for example data logging each time an SCS device is used and at what stimulation level in terms of electrical pulse width and pulse frequency as well as control signals issued to the SCS device to optimize delivery of pain relief. These clinical data can then be accessed by health care staff accessing the data center using suitably designed software applications. Treatment plans and, if necessary, interventions can then be decided upon based on analysis of these clinical data. The analysis may be expert analysis by a health care professional or computer-automated analysis with a software program, e.g., running on computing resource at a neuro-service data center, or a combination of both.

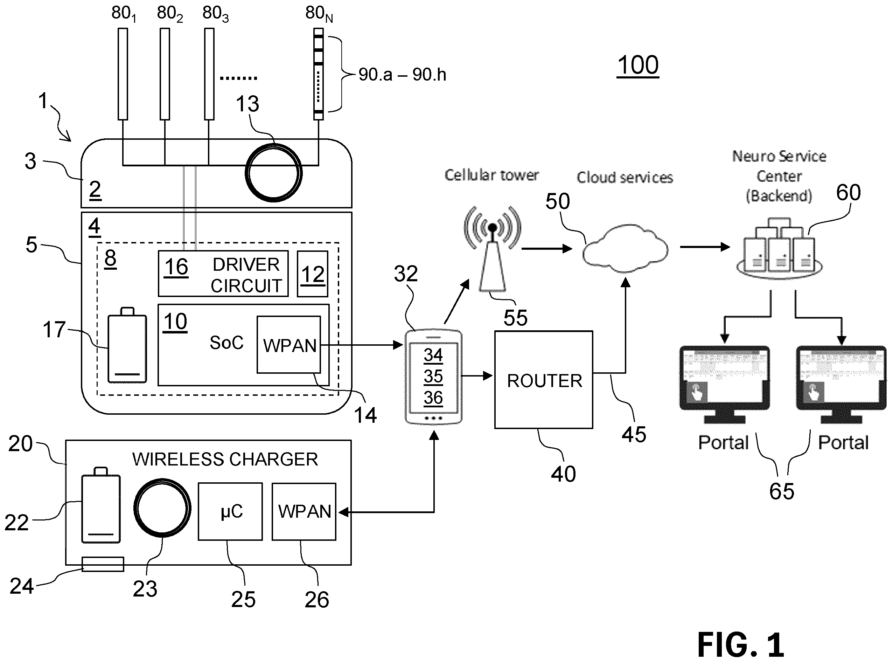

Figure 1 shows a standard architecture of a MDCS 100 for communication between an IMD 1 implanted in a patient and a remotely located data center 60, which is accessible to health care staff using suitable application software. The patient is provided with a patient remote controller 32 (hereinafter patient remote) loaded with a suitable application (so-called app) providing the patient with a user interface to interact with the IMD 1.

The IMD 1 comprises a header 2 and a can 4 which have respective outer casings 3 and 5. The header casing 3 is typically non-metal, e.g. a hard plastics material, epoxy or ceramic. The can casing is made of a biocompatible metal, typically titanium or a titanium alloy. The can 4 includes therapeutic components for patient treatment, such as an SCS driver circuit 16 (or another IMD stimulator) and optionally an SCS sensing circuit for sensing ECAP signals (not shown). The can 4 also contains an electronics unit 8 including a system-on-a-chip (SoC) 10 with control electronic components for controlling the IMD 1, e.g., a microcontroller. One task of the SoC 10 is to deliver control signals to the electrode driver circuit 16 according to a treatment plan. The SoC 10 also includes a wireless personal area network (WPAN) transceiver 14 for external wireless communication, e.g., to the patient remote controller 32 as illustrated. A rechargeable battery 17 is provided for powering the electronics unit 8 and for providing power to the driver circuit 16. The rechargeable battery 17 is wirelessly charged through receive coils 13 which are illustrated placed in the header 2 but in other embodiments are placed in the can 4 or in a separate non-metal housing attached to the can (not shown).

One or more temperature sensors 12 are provided for measuring temperature at locations in the IMD 1 that are expected to become hottest during wireless charging. These locations may include the proximal side of the metal casing 5 (i.e. the side of the casing which is closest to the charger coil during charging), the receive coils 13 and the rechargeable battery 17.

A wireless charger 20 is provided for charging the rechargeable battery 17 of the IMD 1 when the IMD 1 is implanted in a patient. The charger 20 allows charging to take place in a contactless manner by resonant induction. For charging, the charger 20 is placed on the patient's skin adjacent the implanted IMD 1. More specifically, the charger 20 is provided with transmit coils 23 which are matched to the receive coils 13 in the IMD 1. The charger 20 is itself provided with an energy source 22, here a rechargeable battery 22, or multiple rechargeable batteries, as well as an external power connection 24, for example an external power jack, to power the charger 20 and recharge the rechargeable battery 22 from mains power or other external power. In principle, non-rechargeable batteries 22 could be used instead. Another option for charging the rechargeable battery 22 is to do so wirelessly by placing the charger 20 on a mains-powered charging pad. Another option is for the charger 20 to rely on a power cord for its energy supply and not include a battery. Charger operation is controlled by a controller 25, such as a microcontroller. The charger also includes a WPAN transceiver 26 for external communication, for example with the patient remote controller 32 as illustrated. Temperature signals or values obtained by IMD temperature sensors 12 are transmitted to the charger 20, for example via transmission from the IMD 1 directly to the charger 20, or with WPAN transceiver 14 to the patient remote controller 32 and from the patient remote controller 32 to the charger 20. Another option (not illustrated) is to provide a direct near-field communication link between the IMD 1 and charger 20 over which temperature readings from the IMD 1 are communicated directly to the charger 20.

The IMD header 2 is attached to a lead arrangement comprising multiple leads $80_1$ to $80_N$ with each lead incorporating multiple electrodes 90.a to 90.h. The leads 80 attach to the header via ports (not shown) provided in the header 2. In the illustrated example, there are multiple leads but in other examples there may be only one lead. The electrode driver circuit 16 provides a suitable drive current to each of the electrodes 90.a to 90.h according to a set of control parameters that follow a treatment plan as controlled by the SoC 10.

The patient remote 32 may, for example, be a smartphone with wireless transceivers 34, 35, 36 respectively for WPAN (e.g. BLE), LPWAN, cellular (e.g., 4G/LTE/5G) and WLAN communication. If a smartphone is used, this is a smartphone that is possessed, e.g., owned, by the patient in which the IMD 1 is implanted and on which a software application ('app') is installed. The app is then a so-called Software as a Medical Device (SaMD) which is defined by the United States Food and Drug Administration (FDA) as software intended to be used for one or more medical purposes that perform these purposes without being part of a hardware medical device.

The cellular transceiver 35 and WLAN transceiver 36 provide two different data communication paths for the patient remote 32 to upload clinical data from the IMD 1 via an internet connection 50 to a remotely located data center 60 (hereinafter also called "backend 60") acting as repository for storage of clinical data and/or as a host for the services, for example a neuro-service data center. The remote's WLAN transceiver 36 can upload data to the backend 60 via a router 40 and an outer internet connection 45 using a wired internet connection 50. The internet connection 50 may provide access to one or more distributed networks and/or cloud services. The remote's cellular transceiver 35 can upload data to the backend 60 via one or more cellular network base stations 55 (cellular towers), for example LPWAN-capable cellular network base station. In some cases, instead of a public communication network, a dedicated point-to-point transmission, e.g., via a dedicated telephone line, may be provided for uploading clinical data. In all these scenarios, uploading of clinical data from the IMD 1 to the backend 60 takes place via the intermediary of the patient remote 32, the latter thereby acting as a relay device.

Health care staff, such as health care professionals (HCPs), clinical specialists and representatives and remote care team members have access to the backend 60 via suitable portals 65 with the aid of a software application running on the backend 60 and/or the portal 65 to provide the necessary user interfacing, diagnostics and so forth. At least one portal 65 is provided by at least one workstation for health care staff to access a data center, e.g., the backend 60. Analysis and diagnostic software may also be run at the backend 60 to analyze clinical data from individual patients or groups of patients.

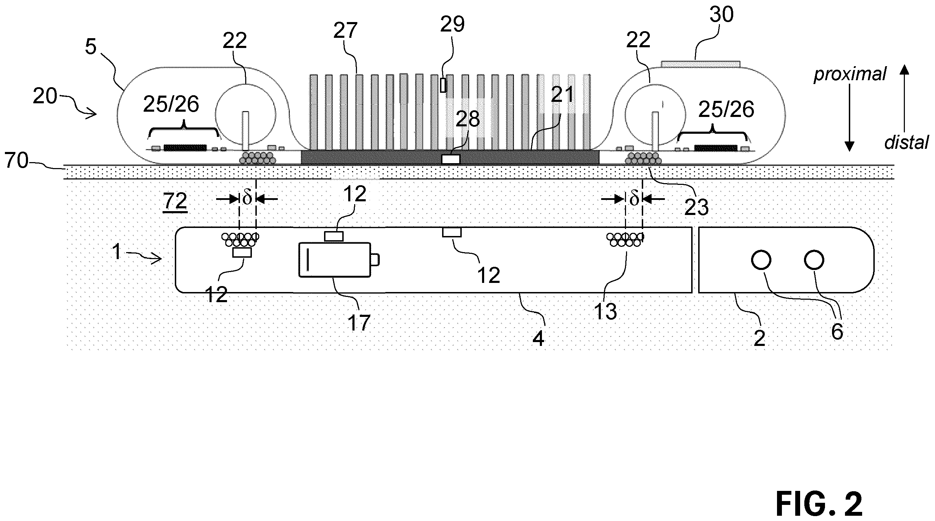

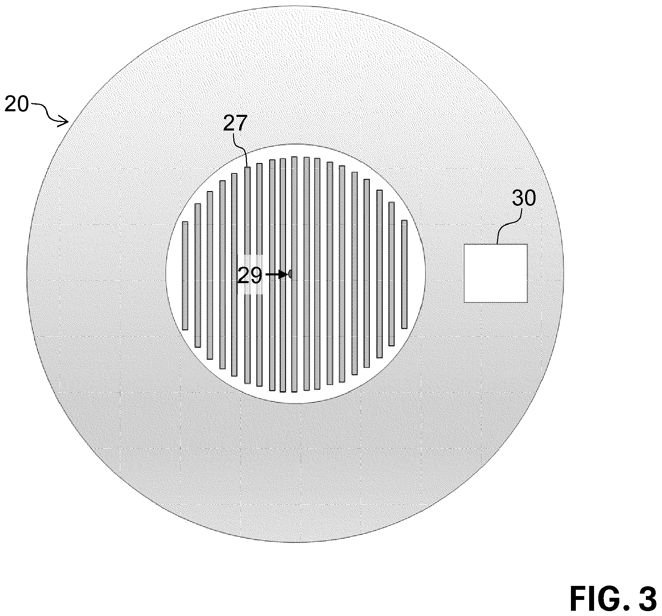

Figures 2 and 3 are schematic cross-sectional and plan views of a charger 20 embodying the invention, with the charger 20 being shown in position for wirelessly charging a subcutaneously implanted IMD 1. The IMD 1 is embedded in the patient's tissue 72 beneath the patient's skin 70. The IMD header 2 and can 4 are shown as are the header's lead attachment ports 6.

The charger 20 is shown with its transmit coils 23 aligned with the IMD's receive coils 13, where the alignment is illustrated as not being perfect, having a lateral offset, $\delta$. A value $\delta = 0$ would correspond to perfect alignment. The receive coils 13 are shown as being accommodated in the can 4 but they may be in the header 2 or a separate housing as previously mentioned.

During charging of an implanted IMD 1, the charger 20 is arranged with a proximal surface in contact with the patient's skin over the implanted IMD 1.

At least an area portion of the proximal surface is actively cooled with a cooling device or cooler 21. A suitable cooling device is a thermoelectric cooler uses the Peltier effect. A thermoelectric cooler has no moving parts, makes no noise, and is compact. A thermoelectric cooler is constructed as a sandwich in which semiconductor pillars arranged alternatingly p-type and n-type are arranged between two plates. When DC biased the semiconductor pillars transfer heat from one plate to the other, i.e., from a cold plate to a hot plate. The cold plate is arranged on the proximal surface of the charger 20 so that it contacts and cools the patient's skin. The Peltier effect occurs when DC current is passed through multiple junctions in series of a p-doped semiconductor to metal to an n-doped semiconductor to metal and back to p-doped semiconductor. Wherever the electrons making up the current transition from an p-doped semiconductor to metal to an n-doped semiconductor, the electrons move up in energy levels, and the electrons obtain the energy they need to make this transition by absorbing heat. Likewise, whenever the electrons transition from an n-doped semiconductor to metal to a p-doped semiconductor the electrons drop in energy levels, and the electrons give off the excess energy in the form of heat. A thermoelectric cooler is made by putting many of these junctions in series, and arranging the junctions where heat is absorbed to be on one side of a structure (the cooled or cold plate), and junctions where excess heat is given off to be on the other side of a structure (the hot plate). The thermoelectric cooler 21 is a heat pump, moving heat from the cold side to the hot side. The hot side needs a mechanism to radiate its heat away.

The cooler 21 acts to cool the skin and the tissue immediately beneath the skin in the region of the implanted IMD. The cooler therefore allows more recharging power than would otherwise be possible to be transmitted by the transmit coil 23 to the receive coil 13, which provides for faster charging of the IMD rechargeable battery 17.

Cooling fins 27 are attached to the hot plate of the thermoelectric cooler to aid heat dissipation into the air.

Another option for carrying away the excess heat from the hot plate (not shown) is to use a belt by which the charger is strapped to the patient and held in position for charging over the implanted IMD. The belt is made of or includes thermally conductive material so that heat can dissipate lengthways along the belt away from the charger 20. In one example, the belt is made of a thermally conductive fabric, which can be made (for example) from silicone, braded stainless steel or a fabric such as nylon or cloth, with conductive metal threads woven in (e.g. copper, stainless steel, or silver).

The charging process is controlled taking account of temperature readings provided by one or more temperature sensors placed at locations on the charger and the IMD where the temperature is expected to be highest and lowest during charging.

The charger 20 is provided with a temperature sensor 28 arranged on the cold plate of the cooler 21 in order to measure skin temperature during charging. Optionally multiple temperature sensors 28 may be arranged at different locations on the cooling plate to take account of lateral temperature variation. The charger 20 is also provided with a temperature sensor 29 arranged on the cooling fins 27. Optionally multiple temperature sensors 29 may be arranged on different fins to take account of lateral temperature variation. The fin temperature sensor(s) are arranged near the top(s) of the fins where to make sure the fins do not become a burn hazard for the patient or a care worker or a fire hazard.

The IMD 1 is also provided with temperature sensors 12 located where it can monitor the temperature of the part of the surface of the IMD 1 in contact with tissue which is expected to become hottest during charging. In one embodiment, the temperature sensor is not directly monitoring the hottest section of the surface of the IMD 1 (because, for example, it is not practical to place a temperature sensor in that exact location) but instead indirectly monitoring that temperature by monitoring a different location and scaling the temperature appropriately. A temperature sensor 12 is arranged on the proximal side of the metal casing of the can 4, which is the closest part of the metal casing to the transmit coil 23 so will be heated the most from eddy currents. A further temperature sensor 12 is arranged on the proximal side of the rechargeable battery, which has a metal jacket that is also prone to eddy current heating during charging. A still further temperature sensor 12 is arranged adjacent the receive coil. Temperature signals or values obtained by IMD temperature sensors are transmitted to the charger, for example via transmission from the IMD with wireless personal area network (WPAN) transceiver to the charger, or directly via a near-field communication link established between the IMD and the charger.

The charger controller therefore takes account of the temperature readings to control charging to ensure: the thermal does complies with ISO 14708-3:2017 (or some other specified standard or criterion); the fins do not exceed a maximum temperature, so that the fins cannot burn a person or cause discomfort if touched on the distal side of the charger; the cold plate and/or the skin temperature does not fall below a minimum temperature to avoid the skin being cooled excessively to cause discomfort or numbness; the skin is not heated excessively, e.g., at no time exceeds 43°C; and no part of the IMD surface in contact with tissue exceeds a certain temperature, e.g., at no time exceeds 43°C. For example, the controller may seek to maintain the skin temperature at about 15°C, which is about 5°C below ambient temperature.

In the preferred embodiment there is one or more temperature sensors (e.g. thermistors) 28, 29 incorporated into the charger 20. In one embodiment there is a temperature sensor 28 on the cooling surface of the thermoelectric cooler. Control circuitry (50) uses input from the temperature sensor 29 to ensure that the temperature of the thermoelectric cooler in contact with skin says within a certain range (not cold enough to be uncomfortable for patient). In another embodiment, there is a temperature sensor 29 incorporated into the cooling fins 27. Control circuitry (50) makes sure that the cooling fins 27 do not get uncomfortably hot to touch. Should the cooling fins 27 get too hot or should the skin contact surface get too cold, the control circuitry (50) reduces the power going to the thermoelectric cooler 21, and therefore reduces the temperature difference between the cold plate and the hot plate of the thermoelectric cooler. Power to the thermoelectric cooler 21 can be reduced by reducing instantons power, duty cycling the thermoelectric cooler, or some other means (such as a combination of reduced instantons power and duty cycling).

A suitable temperature set point for the cold plate of the thermoelectric cooler and/or the cooling fins can be set during manufacture. A suitable temperature may be based on patient comfort rather than actual danger. For the cold plate, this is likely about 15° C (~5° C below room temperature). For the hot side, e.g., at the hot plate or on the cooling fins, this is likely about 43° C (~23°C above room temperature). The physician, other health care professional, or the patient may be given the ability to set these temperatures, at least within certain safe ranges. In general, the lower the set point temperature for the cold plate, the faster the charging that can be achieved. Temperature set point adjustment may take place via an app on the patient remote.

A patient can set the cold plate temperature to a lower value for faster charging, or a higher value if their skin is sensitive to the cold to avoid discomfort. However, if the cold plate temperature is set to be cooler without allowing the cooling fins to get hotter, then no additional cooling will result because the power applied to the thermoelectric cooler will be reduced by the controller to avoid exceeding the temperature set point for the cooling fins. In general, both these temperature set points need to be changed in tandem to allow the system to withdraw heat from the IMD and tissue more rapidly.

The charger may also include a visual indicator 30, such as a display and/or indicator lights, to convey information to the patient, for example on the charging status of the charger, the progress of the charging process, and the charger configuration parameters. A useful indication is for alignment of the transmit and receive coils, which may be binary (aligned/not aligned) with red and green lights, or more a graduated scale showing how well aligned the coils are. Another useful indication is charging level (e.g. with bars or by displaying a percentage value) and/or a countdown of expected remaining charging time to full charge. Useful configuration parameters include the temperature set point values, such as desired skin temperature, for example expressed as a number of degrees below body temperature or ambient temperature. The charge status of the charger batteries 22 may also be shown. Equivalent audible indicators may also be provided, which may for example be useful if the patient is aligning the charger on their back or somewhere else where there is no line of sight to the IMD.

In use, when the IMD is becoming too hot due to charger induced eddy currents on the surface of the IMD, the charger controller 25 can intervene to reduce charging power. Any suitable control algorithm may be used, for example PID (Proportional - Integral - Derivative) control.

With the actively cooled charger proposed, the thermal gradient from the surface of the IMD to the cooled area of the skin is increased. As a consequence, heat will flow away more rapidly from the IMD towards the cooled surface of the skin that is in contact with the cold plate of the thermoelectric cooler. For example, the thermal gradient increases by more than a factor of three if the skin is cooled to 15°C providing three times the cooling power. With the proposed active cooling, the need to reduce charging power, or even pause the charging, will occur less often and may even be eliminated in some cases. Charging times are therefore correspondingly reduced.

The charger 20 may itself be a rechargeable system as illustrated, being powered by its own rechargeable batteries 22. This allows the charger to be a wireless unit and therefore more convenient for the patient to use. In this embodiment the charger incorporates rechargeable batteries 22 that power it during IMD recharging. It is important that the batteries in the charger be large enough to not only adequately charge the IMD, but also to provide the power to the cooling system 21 during the charging period. Thermoelectric coolers 21 typically require several Watts of power to operate, so this must be considered when specifying the batteries 22.

During a charging cycle, the charger controller 25 may be configured to selectively power the thermoelectric cooler 21 depending on the charge state of the charger batteries 22, so that charger battery power is preserved for charging activity. The charger 20 has two main power consumption components; power for the cooler and power to energize the transmit coils to recharge the IMD battery 17. To ensure completion of a charging cycle, the charger controller is configured to stop powering the cooler when the remaining power in the charger batteries falls below the power needed to energize the transmit coils to complete the charging cycle plus some margin to ensure that the charger batteries do not fully discharge. That way, the charging cycle can successfully complete with the IMD battery fully charged, even though this will likely take longer owing to the lack of cooling. The charger controller can take account of this when planning a charging cycle that is about to be initiated or on an ongoing basis during a charging cycle, so that cooling is switched off part way through the charging cycle.

Reference numerals

| 1 | implantable medical device (IMD) |
|---|---|
| 2 | header |
| 3 | header outer casing, e.g., ceramic, plastics, epoxy |
| 4 | can |
| 5 | charger outer casing, e.g. titanium or titanium alloy |
| 6 | lead attachment ports |
| 8 | electronics unit |
| 10 | system-on-a-chip (SoC) |
| 12 | temperature sensors |
| 13 | receive coils |
| 14 | wireless personal area network (WPAN) transceiver |
| 16 | driver circuit, e.g., SCS device with attached leads |
| 17 | rechargeable battery |
| 20 | charger |
| 21 | thermoelectric cooler |
| 22 | charger rechargeable battery |
| 23 | charger transmit coils |
| 24 | charger external power connection |
| 25 | charger controller, e.g., microcontroller |
| 26 | charger WPAN transceiver |
| 27 | cooling fins |
| 28 | cooler/skin contact surface temperature sensor |
| 29 | cooling fin temperature sensor |
| 30 | charger visual indicator, e.g., display |
| 32 | patient remote controller (patient remote) |
| 34 | smartphone wireless personal area network (WPAN) transceiver |
| 35 | smartphone cellular transceiver |
| 36 | smartphone wireless local area network (WLAN) transceiver |
| 40 | router |
| 45 | router internet connection |
| 50 | internet connection |
| 55 | cellular network base station (cellular tower) |
| 60 | remotely located data center (backend) |
| 65 | portal |
| 70 | patient's skin |
| 72 | patient's tissue |
| $80_1$ to $80_N$ | leads |
| 90.a to 90.h | electrodes |
| 100 | medical device communication system (MDCS) |

**Claims**

1. A charger (20) for wirelessly charging an implantable medical device, IMD (1) implanted in a patient, the charger comprising:

   an electrical energy source (22, 24);
   transmit coils (23) arranged to generate an electromagnetic field for wirelessly charging an implanted IMD (1); and
   a cooling device (21) powered by the electrical energy source and arranged to cool a surface portion of the charger that is configured to be in contact with the patient's skin during wireless charging.

2. The charger of claim 1, wherein the cooling device is a thermoelectric cooler operating by the Peltier effect, the thermoelectric cooler having a cold plate and a hot plate, the cold plate forming said surface portion.

3. The charger of claim 1 or 2, further comprising cooling fins (27) arranged to extract heat from the cooling device.

4. The charger of claim 3, further comprising at least one temperature sensor (29) arranged to measure temperature of at least one of the cooling fins.

5. The charger of any one of the preceding claims, further comprising at least one temperature sensor (28) arranged to measure temperature of said charger surface portion.

6. The charger of any one of the preceding claims, further comprising a controller (25) loaded with machine readable instructions to control the charging of an IMD (1).

7. The charger of claim 6, when dependent on claim 4 or 5, wherein the charging of an IMD (1) is controlled by the controller according to the machine-readable instructions dependent on the temperatures measured by the temperature sensors.

8. The charger of claim 6 or 7, further comprising a wireless transceiver (26) operable to receive IMD (1) temperature data from the IMD (1) being charged, wherein the charging of the implanted IMD (1) is controlled by the controller according to the machine-readable instructions dependent on the IMD (1) temperature data.

9. The charger of any one of the preceding claims, wherein the electrical energy source is selected from one or more of the group: rechargeable battery (22); non-rechargeable battery (22); and external electrical power connection (24).

**FIG. 1**

**100**

Neuro Service Center (Backend) 60

Portal 65 Portal

Cloud services 50

Cellular tower 55

ROUTER 45 40

32 34 35 36

90.a – 90.h

$80_N$ $80_3$ $80_2$ $80_1$ 13

DRIVER CIRCUIT 12 16

SoC 10 WPAN 14

8 17 4 2 3 1 5

WIRELESS CHARGER 20

μC 25 WPAN 26

22 23 24

9

**FIG. 2**

**FIG. 3**

Europäisches
Patentamt

European
Patent Office

Office européen
des brevets

## EUROPEAN SEARCH REPORT

**Application Number**

EP 24 19 4442

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 7 505 816 B2 (MEDTRONIC INC [US]) 17 March 2009 (2009-03-17) * columns 7-19; claim 1; figures 3,13,17; tables ,3-7 * | 1-9 | INV. A61N1/378 H02J50/10 |
| X | US 10 258 804 B2 (MEDTRONIC INC [US]) 16 April 2019 (2019-04-16) * columns 5-9; figure 1 * | 1 | |
| A | US 2019/134408 A1 (VON NOVAK III WILLIAM HENRY [US] ET AL) 9 May 2019 (2019-05-09) * claim 1; figures 1-2 * | 1-9 | |
| A | US 2024/097499 A1 (FRIED ANDREW THOMAS [US] ET AL) 21 March 2024 (2024-03-21) * paragraphs [0051], [0148] * | 1-9 | |

TECHNICAL FIELDS
SEARCHED (IPC)

A61N
H02J

The present search report has been drawn up for all claims

| Place of search | Date of completion of the search | Examiner |
|---|---|---|
| Munich | 30 September 2024 | Gentil, Cédric |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 19 4442

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2024

| Patent document cited in search report | | Publication date | Patent family member(s) | | Publication date |
|---|---|---|---|---|---|
| US 7505816 | B2 | 17-03-2009 | AT | E443538 T1 | 15-10-2009 |
| | | | EP | 1885448 A1 | 13-02-2008 |
| | | | US | 2006247738 A1 | 02-11-2006 |
| | | | WO | 2006119098 A1 | 09-11-2006 |
| US 10258804 | B2 | 16-04-2019 | US | 2018126177 A1 | 10-05-2018 |
| | | | WO | 2018084978 A1 | 11-05-2018 |
| US 2019134408 | A1 | 09-05-2019 | US | 2019134408 A1 | 09-05-2019 |
| | | | WO | 2019094200 A1 | 16-05-2019 |
| US 2024097499 | A1 | 21-03-2024 | US | 2024097499 A1 | 21-03-2024 |
| | | | WO | 2022182828 A1 | 01-09-2022 |

EPO FORM P0459

For more details about this annex : see Official Journal of the European Patent Office, No. 12/82